(19) Europäisches Patentamt
European Patent Office
Office européen des brevets

(11) Veröffentlichungsnummer : **0 070 463**
**B1**

(12) **EUROPÄISCHE PATENTSCHRIFT**

(45) Veröffentlichungstag der Patentschrift : **09.01.85**

(51) Int. Cl.⁴ : **C 07 C 51/58**, C 07 C 53/42, C 07 C 53/50

(21) Anmeldenummer : **82106112.4**

(22) Anmeldetag : **08.07.82**

# (54) Verfahren zur Herstellung von Säurehalogeniden.

(30) Priorität : **18.07.81 DE 3128445**

(43) Veröffentlichungstag der Anmeldung : **26.01.83 Patentblatt 83/04**

(45) Bekanntmachung des Hinweises auf die Patenterteilung : **09.01.85 Patentblatt 85/02**

(84) Benannte Vertragsstaaten : **AT BE CH DE FR GB IT LI NL**

(56) Entgegenhaltungen :
**DE-A- 2 648 134**
**US-A- 2 378 048**
**US-A- 2 411 982**
**US-A- 2 580 070**
**US-A- 3 471 557**
**ANNALEN DER CHEMIE Band 625, 1959 Weinheim-Bergstrasse A.T. BALABAN et al. "Die Reaktion der Alkane mit Kohlenoxyd" Seiten 66 bis 73**

(73) Patentinhaber : **BAYER AG**
**Konzernverwaltung RP Patentabteilung**
**D-5090 Leverkusen 1 Bayerwerk (DE)**

(72) Erfinder : **Blank, Heinz Ulrich, Dr.**
**Am Geusfelde 35**
**D-5068 Odenthal (DE)**
Erfinder : **Wolters, Erich, Dr.**
**Streffenweg 22**
**D-5162 Niederzier (DE)**

**EP 0 070 463 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Verfahren zur Herstellung von organischen Säurehalogeniden durch Reaktion von Alkylhalogeniden mit Kohlenmonoxid.

Aus Justus Liebigs Ann. *625*, 66 ff. (1959) ist es bekannt, daß tertiäre Alkylhalogenide in Gegenwart von etwa stöchiometrischen Mengen von Aluminiumchlorid oder Eisen-(III)-chlorid bei 150 bar mit Kohlenmonoxid reagieren, wobei in schlechter Ausbeute t-Butyl-isobutenylketon neben wenig Pivalinsäure erhalten wurde.

Nach US 2.580.070, Spalte 1 Zeilen 12-21, ist es ganz allgemein nicht möglich, Säurechloride in substantiellen Ausbeuten mit irgendeiner Methode herzustellen, welche die Verwendung von Alkylchloriden und Kohlenmonoxid als Ausgangsstoffe und Aluminiumchlorid als Katalysator einschließt.

Aus US 2.580.070 ist ferner bekannt, t-Butylchlorid und t-Amylbromid mit äquimolaren Mengen Bortrifluorid bei 0 °C und einem Kohlenmonoxiddruck von 700 atm zum zugehörigen Pivaloylchlorid bzw. α,α-Dimethylpropionylbromid umsetzen, wobei t-Butylchlorid trotz des hohen CO-Druckes maximal mit 49 Mol-% Umsatz zur Reaktion gebracht werden konnte. Zudem ist eine aufwendige Aufarbeitung für die Wiedergewinnung oder Beseitigung des $BF_3$ erforderlich. Führt man die Reaktion zwischen t-Butylchlorid und Kohlenmonoxid mit 10 % Bismuthtrichlorid oder mit 2 % Zinntetrachlorid bei Temperaturen von 50 und 150 °C durch, so betragen die Umsätze nur noch 5 und 0 %, wobei kein Pivaloylchlorid isoliert werden konnte.

Es wurde nun ein Verfahren zur Herstellung von Säurechloriden der Formel

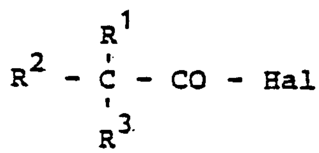

(I)

in der

$R^1$, $R^2$ und $R^3$ unabhängig voneinander verzweigtes oder unverzweigtes, gegebenenfalls durch Halogen substituiertes Alkyl oder Cycloalkyl bedeuten,

wobei weiterhin zwei der Reste $R^1$, $R^2$ und $R^3$ gemeinsam mit dem C-Atom, das sie subtituieren, ein Ringsystem bilden können und

Hal für ein Halogenatom steht,

durch Reaktion von Alkylhalogeniden der Formel

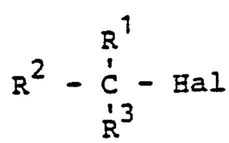

(II)

in der $R^1$, $R^2$, $R^3$ und Hal die genannte Bedeutung haben, mit Kohlenmonoxid bei erhöhtem Druck, bei erniedrigter bis erhöhter Temperatur gefunden, das dadurch gekennzeichnet ist, daß die Umsetzung in Gegenwart von 0,005 bis 0,2 Mol Aluminiumchlorid und/oder Eisenchlorid pro Mol Alkylhalogenid und gegebenenfalls in Gegenwart einer weiteren Brönsted- oder Lewissäure und gegebenenfalls in Gegenwart eines Lösungsmittels durchgeführt wird.

Das Säurehalogenid kann aus dem Reaktionsgemisch isoliert werden oder direkt im Reaktionsgemisch umgesetzt werden.

Als Halogenatom sei beispielsweise ein Fluor-, Chlor- oder Bromatom, bevorzugt ein Chlor- oder Bromatom, besonders bevorzugt ein Chloratom, genannt.

Als verzweigtes oder unverzweigtes Alkyl sei ein solches mit beispielsweise 1 bis 20, bevorzugt 1 bis 10, besonders bevorzugt 1 bis 3, Kohlenstoffatomen genannt, wie beispielsweise Methyl, Ethyl, Propyl, Isopropyl, Butyl, Isobutyl, t-Butyl, Amyl, Isoamyl, t-Amyl, Hexyl,, Isohexyl, Octyl, Isooctyl, Decyl, Isodecyl, Dodecyl, Isododecyl, Stearyl, Isostearyl, Eicosyl oder Isoeicosyl. Die unverzweigten Alkylreste sind bevorzugt.

Als Halogenalkyl sei beispielsweise ein einfach oder mehrfach durch Halogen substituiertes Alkyl, wie beschrieben, genannt, wie Chlormethyl, Dichlormethyl, Trichlormethyl, Brommethyl, Dibrommethyl, Tribrommethyl, Fluormethyl, Difluormethyl, Trifluormethyl, einfach oder mehrfach chloriertes, bromiertes oder fluoriertes Ethyl, Propyl, Butyl, Hexyl, Octyl, Decyl, Dodecyl, Stearyl oder Eicosyl.

Als Cycloalkyl sei ein gegebenenfalls Methyl- oder Ethyl-substituierter cycloaliphatischer Rest von beispielsweise 3 bis 20, bevorzugt 4 bis 12, besonders bevorzugt 5 bis 6 Ringkohlenstoffatomen genannt, wie beispielsweise Cyclopropyl, Cyclobutyl, Cyclopentyl, Methyl-cyclopentyl, Ethyl-cyclopentyl, Cyclohexyl, Methylcyclohexyl, Ethyl-cyclohexyl, Cycloheptyl, Cyclooctyl, Cyclododecyl oder Cycloeicosyl.

Als Halogencycloalkyl sei beispielsweise ein einfach oder mehrfach chloriertes, bromiertes oder

fluoriertes Cycloalkyl, wie beschrieben, genannt, wie Chlorcyclopentyl, Chlorcyclohexyl, Bromcyclopentyl oder Bromcyclohexyl.

Weiterhin können 2 der Reste $R^1$, $R^2$ und $R^3$ gemeinsam mit dem C-Atom, das sie substituieren, ein Ringsystem bilden. Ein solches Ringsystem hat beispielsweise 3 bis 12, bevorzugt 5 bis 6 C-Atome. Beispiele für solche Ringsysteme sind : Cyclopropan, Cyclobutan, Cyclopentan, Cyclohexan, Cycloheptan, Cyclooctan, Cyclododecan.

In bevorzugter Weise werden im erfindungsgemäßen Verfahren Alkylhalogenide der Formel

$$R^5 - \underset{R^6}{\overset{R^4}{C}} - Hal \qquad (III)$$

eingesetzt, in der

$R^4$, $R^5$ und $R^6$ unabhängig voneinander unverzweigte Alkylreste bedeuten und

Hal die oben genannte Bedeutung hat.

In den Formeln (I), (II) und (III) sind als Halogenide die Bromide oder Chloride bevorzugt und die Chloride ganz besonders bevorzugt.

Beispiele für erfindungsgemäß einsetzbare Alkylhalogenide sind : t-Butylchlorid, t-Amylbromid, 2-Chlor-2-methylhexan, 2-Brom-2-ethylpentan, 2-Chlor-2-propylhexan, 1-Chlor-1-methylcyclopropan, 1-Chlor-1-methylcyclopentan, 1-Chlor-1-methylcyclohexan, 1-Brom-1-methylcyclohexan, 1-Brom-1-methylcyclododecan, 1,2-Dichlor-2-methyl-propan, 1,2-Dichlor-2-chlormethyl-butan, 1,2,3-Trichlor-3-methyl-butan, 1,2-Dibrom-2-methyl-propan, 2-Chlor-2-(2-chlorcyclohexyl)-propan, 1,2-Dichlor-2-(2-chlorcyclohexyl)-butan, 1,2,3-Trichlor-2-chlormethyl-propan, 1,2,3-Trichlor-2-methyl-propan.

Das erfindungsgemäße Verfahren wird bei einem Kohlenmonoxiddruck durchgeführt, der in weiten Grenzen schwaken kann, beispielsweise von 5 bis 1 000 bar. Das Verfahren kann auch oberhalb des genannten Bereiches durchgeführt werden und ist nach oben hin nur durch den für angemessen erachteten technischen Aufwand begrenzt. Unterhalb von 5 bar werden die Umsätze merklich geringer. In bevorzugter Weise wird ein CO-Druck von 25 bis 250 bar, besonders bevorzugt von 50 bis 150 bar angewendet.

Als Temperaturbereich für die Durchführung des erfindungsgemäßen Verfahrens sei beispielsweise − 20 bis + 100 °C, bevorzugt − 20 bis + 50 °C, besonders bevorzugt − 10 bis + 10 °C und ganz besonders bevorzugt 0 bis + 5 °C genannt. Vielfach nehmen Ausbeute und Selektivität mit steigender Temperatur etwas ab, so daß tiefere Temperaturen im allgemeinen günstiger sind. Es ist jedoch meistens nicht erforderlich, Temperaturen unter − 10 °C anzuwenden. Sofern die Umsetzung in dem besonders bevorzugten Temperaturbereich von − 10 bis + 10 °C, insbesondere 0 bis + 5 °C durchgeführt wird, ist es zweckmäßig, im Anschluß an den Reaktionsschritt eine Nachreaktion bei erhöhten Temperaturen von 10-100 °C, insbesondere 20-50 °C durchzuführen und erst im Anschluß an diesen Nachreaktionsschritt aufzuarbeiten.

Die optimale Temperatur kann in Abhängigkeit vom eingesetzten Alkylhalogenid, vom Katalysatorsystem und vom Lösungsmittel durch einfache Vorversuche leicht ermittelt werden.

Das erfindungsgemäße Verfahren wird mit $AlCl_3$ und/oder $FeCl_3$ als Katalysator und gegebenenfalls in Gegenwart einer weiteren Brönsted- oder Lewis-Säure durchgeführt. Beispielhaft seien hierfür erwähnt : Halogenide der Elemente, besonders der Metalle, der dritten, vierten und fünften Hauptgruppe und der ersten, zweiten, vierten, fünften, sechsten, siebenten und achten Nebengruppe des Periodensystems der Elemente (Mendelejew), beispielsweise Eisen-(III)-bromid, Zinkchlorid, Zinkbromid, Borchlorid, Galliumchlorid, Titantetrachlorid, Antimon-(III)-chlorid, Antimon-(V)-chlorid, Antimon-(III)-bromid oder Antimon-(V)-bromid. Die genannten Halogenid können einzeln oder als Gemisch mehrerer der genannten, jeweils in wasserfreier Form, eingesetzt werden. Diesen Halogeniden können Halogenide von Metallen der ersten und zweiten Hauptgruppe des Periodensystems, wie Lithiumchlorid, Natriumchlorid oder Magnesiumchlorid, zugesetzt werden. Halogenide der genannten Art oder Gemische der genannten Art können gegebenenfalls auf einem Trägermaterial, wie Aluminiumoxid, Kieselgel oder Aktivkohle angewandt werden. Bevorzugt werden Aluminiumchlorid oder Eisen-(III)-chlorid ohne Zusätze, besonders bevorzugt Aluminiumchlorid, angewandt.

$AlCl_3$ und $FeCl_3$ werden erfindungsgemäß in einer Menge von 0,005 bis 0,2 Mol, bevorzugt 0,01 bis 0,1 Mol pro Alkylhalogenid, eingesetzt. Es ist weiterhin möglich, die Reaktion des erfindungsgemäßen Verfahrens in Gegenwart von Halogenwasserstoff, beispielsweise Chlorwasserstoff, in einer Menge von 0,005 bis 2 Mol, bevorzugt 0,01 bis 0,1 Mol, pro Mol Alkylhalogenid, durchzuführen. Größere Mengen an Halogenwasserstoff als die angegebenen sind für das erfindungsgemäße Verfahren unkritisch.

Für den Fall, daß in Gegenwart von Halogenwasserstoff gearbeitet wird, ist es weiterhin möglich, an Stelle der Alkylhalogenide die zugrundeliegenden Olefine einzusetzen, die sodann im Reaktionsgemisch mit Halogenwasserstoff une Kohlenmonoxid erfindungsgemäß umgesetzt werden. Hierzu ist eine mindestens äquimolare Menge Halogenwasserstoff, bezogen auf das Olefin, erforderlich.

Das erfindungsgemäße Verfahren kann mit oder ohne Lösungsmittel durchgeführt werden. Die

Variante ohne Lösungsmittel wird immer dann möglich sein, wenn das eingesetzte Alkylhalogenid bei der gewählten Reaktionstemperatur flüssig ist. Für den Fall, daß ein Lösungsmittel eingesetzt wird sei allgemein ein solches genannt, das für Friedel-Crafts-artige Reaktionen üblich ist. Geeignete organische Lösungsmittel sind beispielsweise halogenierte Kohlenwasserstoffe, wie Dichlorethan, Trichlorethan, Tetrachlorethan, Methylenchlorid, Chlorbenzol, Dichlorbenzol, Trichlorbenzol, weiterhin Schwefelkohlenstoff, Benzolsulfonsäure, Methansulfonsäure, Trifluormethansulfonsäure, Perfluorbutansulfonsäure, Perfluoroctansulfonsäure, Trifluoressigsäure, Alkane, wie Hexan, Octan oder Isododecan oder Nitrobenzol. Geeignete anorganische Lösungsmittel sind beispielsweise $SO_2$, HCl, HF, $H_2SO_4$, $PCl_3$ oder $POCl_3$. Diese Lösungsmittel können einzeln oder in Gemischen eingesetzt werden. Bevorzugte Lösungsmittel sind chlorierte Kohlenwasserstoffe, wie Methylenchlorid, Dichlorethan, Dichlorbenzol und Trichlorbenzol. Die Durchführung des erfindungsgemäßen Verfahrens in Gegenwart eines Lösungsmittels ist bevorzugt. Als Menge des zu verwendenden Lösungsmittels sei beispielsweise 20 bis 500 Vol.-%, bevorzugt 50 bis 250 Vol.-%, besonders bevorzugt 80 bis 150 Vol.-%, bezogen auf das Volumen des eingesetzten Alkylhalogenids, genannt.

Das erfindungsgemäße Verfahren kann beispielsweise wie folgt durchgeführt werden :

In einem V4A-Stahlautoklaven wird das Lösungsmittel mit der katalytischen Menge des Katalysators vorgelegt. Dann wird der gewünschte CO-Druck aufgepreßt und unter gutem Rühren das Alkylhalogenid unverdünnt oder mit Lösungsmittel verdünnt eingepumpt. Durch Nachführen von CO, beispielsweise über ein Reduzierventil, wird der CO-Druck aufrechterhalten. Da die Reaktion im allgemeinen sehr schnell abläuft, kann zügig weiteres Alkylhalogenid eingepumpt werden oder mit Vorteil sogar kontinuierlich gearbeitet werden. Nach kurzem Nachrühren wird kein CO mehr aufgenommen, und die Reaktion kann abgebrochen werden. Das Reaktionsgemisch, das außer dem Lösungsmittel praktisch nur Säurehalogenid und gegebenenfalls etwas restliches Alkylhalogenid enthält, kann entweder in dieser Form zu Folgeumsetzungen des Säurechlorids, beispielsweise zur Herstellung der Säure oder deren Derivate wie Ester, Amide, Peroxide eingesetzt werden oder kann auf reines Säurechlorid aufgearbeitet werden. Dies ist beispielsweise durch Destillation auf einfache Weise möglich, insbesondere dann, wenn ein Lösungsmittel eingesetzt wird, das bezüglich der destillativen Trennung einen günstig gelegenen Siedepunkt hat.

Als Kopfprodukt einer solchen Destillation erhält man nicht umgesetztes Alkylhalogenid, eingesetztes Lösungsmittel und das gewünschte Säurehalogenid, gegebenenfalls bei entsprechend geführter Destillation als bereits in reiner Form vorliegende Fraktionen.

In vorteilhafter und nach den Befunden der Literatur überraschender Weise können die gewünschten Säurehalogenide im erfindungsgemäßen Verfahren mit bis zu 80 %iger Selektivität, in vielen Fällen bis zu 90 %iger Selektivität und in hoher Ausbeute hergestellt werden.

## Beispiele 1-17

In einem 0,71 V4A-Autoklaven werden 185 ml des in der Tabelle angegebenen Lösungsmittels und die in der Tabelle angegebene Menge und Art des Katalysators vorgelegt. Bei der in der Tabelle angebenen Temperatur und dem dort angegebenen CO-Druck werden unter Rühren 185 g (217 ml ; 2,0 mol) t-Butylchlorid innerhalb von 20 Minuten eingepumpt. Der jeweilige CO-Druck wird durch Nachführung von CO über ein Reduzierventil konstant gehalten. Nach der in der Tabelle aufgeführten Nachrührzeit wird kein CO mehr aufgenommen. Der Autoklav wird entspannt, und das Reaktionsgemisch über eine 30 cm-Vigreuxkolonne destilliert. Es werden die in der Tabelle angegebenen Ausbeuten erhalten.

(Siehe Tabelle Seite 5 f.)

(Tabelle)

| Beispiel | Katalysator Art | Katalysator g (Mol%) | T °C | $P_{CO}$ bar | t min | Lösungsmittel | t-Butylchlorid g (% d. Th.) | Pivaloylchlorid g (% d. Th.) | Umsatz % | Selektivität % |
|---|---|---|---|---|---|---|---|---|---|---|
| 1 | $AlCl_3$ | 2,7 (1,0) | 0-3 | 140 | 15 | 1,3,4-Trichlorbenzol | 69,7 (37,7) | 134,9 (56,0) | 62,3 | 89,9 |
| 2 | " | 9,3 (3,5) | " | " | " | " | 33,3 (18,0) | 166,3 (69,0) | 82,0 | 84,2 |
| 3 | " | 26,7 (10,0) | " | " | " | " | 32,o (17,3) | 106,4 (44,2) | 82,7 | 53,4 |
| 4 | " | 9,3 (3,5) | " | 150 | " | " | 22,2 (12,0) | 171,1 (71,0) | 88,0 | 80,0 |
| 5 | " | " | " | 250 | " | " | 18,5 (10,0) | 176,2 (73,1) | 90,0 | 81,2 |
| 6 | " | " | " | 90 | 30 | " | 34,8 (18,8) | 146,0 (60,5) | 81,2 | 74,6 |
| 7 | " | " | " | 50 | 45 | " | 75,8 (41,o) | 92,4 (38,3) | 59,0 | 65,o |
| 8 | " | " | " | 10 | 15 | " | 120.3 (65,0) | 17,7 (7,3) | 35.0 | 21,0 |
| 9 | " | " | " | 5 | 15 | " | 152,6 (82,4) | 2,4 (1,0) | 17,6 | 5,7 |
| 10 | " | " | 10 | 100 | 30 | " | 49,4 (26,7) | 129,3 (53,7) | 73,3 | 73,2 |
| 11 | " | " | 20 | " | " | " | 62,3 (33,7) | 24,0 (10,0) | 66,3 | 15,0 |
| 12 | " | " | 50 | " | " | " | 115,8 (62,6) | 8,2 (3,4) | 37,4 | 9,1 |
| 13 | " | " | -10 | " | " | 1,2-Dichlorbenzol | 55,3 (29,9) | 144,1 (59,8) | 70,1 | 85,3 |
| 14 | " | " | 0-3 | 140 | 15 | Methylenchlorid | 37,4 (20,2) | 164,6 (68,3) | 79,8 | 85,6 |
| 15 | $FeCl_3$ | 11,4 (3,5) | " | " | " | Trichlorbenzol | 77,7 (42,0) | 86,8 (36,0) | 58,0 | 62,1 |
| 16 | $AlCl_3$ | 1,4 (o,5) | " | 150 | " | " | 110,0 (59,5) | 89,6 (37,2) | 40,5 | 91,8 |
| 17 | " | 53,3 (20,0) | -10-0 | " | " | Dichlorbenzol | 16,3 (8,8) | 63,1 (26,2) | 91,2 | 28,8 |

Beispiel 18

In einem 2,7 l V4A-Autoklaven werden 833 ml (1 205 g) 1,2,4-Trichlorbenzol und 42,00 g (0,31 mol) $AlCl_3$ vorgelegt. Unter Rühren wird bei einer Temperatur von 5 °C und einem CO-Druck von 150 bar innerhalb von 10 Minuten 832,5 g (9,0 mol) t-Butylchlorid eingepumpt. Der CO-Druck wird durch Nachführen von CO über ein Reduzierventil auf 150 bar gehalten. Nach 10-minütigem Nachrühren wird für 30 Minuten auf 50 °C aufgeheizt. Dabei steigt der Druck im Autoklaven auf ca. 165 bar an.

Nach dem Abkühlen wird das Reaktionsgemisch destilliert. Es werden erhalten :

110,7 g t-Butylchlorid (13,3 %)
797,1 g Pivaloylchlorid (73,5 %)

Bei einem Umsatz von 86,7 % wird eine Selektivität von 84,8 % erreicht.

Beispiel 19

In einem 0,7 l V4A-Autoklaven werden 185 ml (268 g) Trichlorbenzol und 13,3 g (0,1 mol) $AlCl_3$ vorgelegt. Bei 0-3 °C und 120 bar CO werden unter Rühren 254,0 g (2,0 mol) 1,2-Dichlor-2-methylpropan innerhalb von 20 Minuten eingepumpt. Der CO-Druck wird durch Nachführen von CO über ein Reduzierventil konstant gehalten. Nach 20 Minuten wird kein CO mehr aufgenommen. Der Autoklav wird entspannt und das dunkelgelbe Reaktionsgemisch über eine 30 cm Vigreuxkolonne destilliert. Es werden 50,3 g 1,2-Dichlor-2-methylpropan und 208,6 g Chlorpivaloylchlorid erhalten. Dies entspricht einem Umsatz von 80,2 % und einer Selektivität von 83,9 %.

Beispiel 20

In einem 0,7 l V4A-Autoklaven werden 185 ml (268 g) Trichlorbenzol und 13,3 g (0,1 mol) $AlCl_3$ vorgelegt. Bei 0-3 °C wird bis zu einem Druck von 20 bar HCl eingepumpt. Anschließend werden unter Rühren 181,0 g (2,0 mol) Methallylchlorid eingepumpt. Durch Nachführen von HCl wird der HCl-Druck aufrechterhalten. Es wird 20 Minuten nachgerührt. Dann wird der Druck durch Aufpressen von 120 bar CO auf 140 bar erhöht und durch Nachführen von CO auf dieser Höhe gehalten. Nach einer Stunde wird kein CO mehr aufgenommen. Der Autoklaveninhalt wird destilliert. Es werden erhalten 54,7 g 1,2-Dichlor-2-methylpropan und 198,4 g Chlorpivaloylchlorid. Dies entspricht einem Umsatz von 78,5 % und einer Selektivität von 81,5 %.

**Ansprüche**

1. Verfahren zur Herstellung von Säurechloriden der Formel

$$R^2 - \overset{\displaystyle R^1}{\underset{\displaystyle R^3}{C}} - CO - Hal$$

in der

$R^1$, $R^2$ und $R^3$ unabhängig voneinander verzweigtes oder unverzweigtes, gegebenenfalls durch Halogen substituiertes Alkyl oder Cycloalkyl bedeuten,

wobei weiterhin zwei der Reste $R^1$, $R^2$ und $R^3$ gemeinsam mit dem C-Atom, das sie substituierten, ein Ringsystem bilden können, und

Hal für ein Halogenatom steht,

durch Reaktion von Alkylhalogeniden der Formel

$$R^2 - \overset{\displaystyle R^1}{\underset{\displaystyle R^3}{C}} - Hal$$

in der

$R^1$, $R^2$, $R^3$ und Hal die genannte Bedeutung haben,

mit Kohlenmonoxid bei erhöhtem Druck, bei erniedrigter bis erhöhter Temperatur, dadurch gekennzeichnet, daß die Umsetzung in Gegenwart von 0,005 bis 0,2 Mol Aluminiumchlorid und/oder Eisenchlorid pro Mol Alkylhalogenid und gegebenenfalls in Gegenwart einer weiteren Brönsted- oder Lewissäure und gegebenenfalls in Gegenwart eines Lösungsmittels durchgeführt wird.

2. Verfahren nach Anspruch 1, dadurch gekennzeichnet, daß Aluminiumchlorid eingesetzt wird.

3. Verfahren nach Ansprüchen 1 bis 2, dadurch gekennzeichnet, daß in Gegenwart von 20 bis 500 Vol.-% eines Lösungsmittels, bezogen auf das Volumen des Alkylhalogenids, gearbeitet wird.

4. Verfahren nach Ansprüchen 1 bis 2, dadurch gekennzeichnet, daß in Gegenwart von 50 bis 250 Vol.-% eines Lösungsmittels gearbeitet wird.

5. Verfahren nach Ansprüchen 1 bis 2, dadurch gekennzeichnet, daß in Gegenwart von 80 bis 150 Vol.-% eines Lösungsmittels gearbeitet wird.

6. Verfahren nach Ansprüchen 1 bis 5, dadurch gekennzeichnet, daß als Lösungsmittel ein für Friedel-Crafts- und Friedel-Crafts-ähnliche Reaktionen gebräuchliches Lösungsmittel eingesetzt wird.

7. Verfahren nach Ansprüchen 1 bis 6, dadurch gekennzeichnet, daß als Lösungsmittel ein chlorierter Kohlenwasserstoff eingesetzt wird.

**Claims**

1. Process for the preparation of acid chlorides of the formula

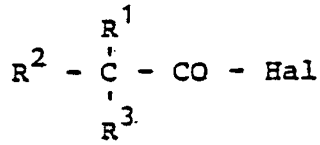

in which

$R^1$, $R^2$ and $R^3$ independently of one another denote branched or unbranched, optionally halogen-substituted alkyl or cycloalkyl,

it furthermore being possible for two of the radicals $R^1$, $R^2$ and $R^3$, together with the C atom on which they are substituents, to form a ring system, and

Hal represents a halogen atom,

by reacting alkyl halides of the formula

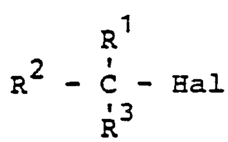

in which

$R^1$, $R^2$, $R^3$ and Hal have the meaning mentioned,

with carbon monoxide under an elevated pressure, and at a reduced to elevated temperature, characterised in that the reaction is carried out in the presence of 0.005 to 0.2 mol of aluminium chloride and/or iron chloride per mol of alkyl halide and, if appropriate, in the presence of a further Brønsted or Lewis acid and, if appropriate in the presence of a solvent.

2. Process according to Claim 1, characterised in that aluminium chloride is used.

3. Process according to Claims 1 to 2, characterised in that the reaction is carried out in the presence of 20 to 500 % by volume of a solvent, based on the volume of the alkyl halide.

4. Process according to Claims 1 to 2, characterised in that the reaction is carried out in the presence of 50 to 250 % by volume of a solvent.

5. Process according to Claims 1 to 2, characterised in that the reaction is carried out in the presence of 80 to 150 % by volume of a solvent.

6. Process according to Claims 1 to 5, characterised in that the solvent used is a solvent which is customary for Friedel-Crafts and Friedel-Crafts-related reactions.

7. Process according to Claims 1 to 6, characterised in that the solvent used is a chlorinated hydrocarbon.

**Revendications**

1. Procédé de préparation de chlorures d'acides de formule

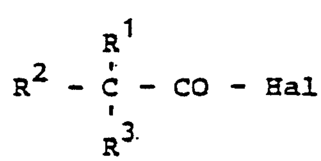

dans laquelle

$R^1$, $R^2$ et $R^3$ représentent chacun, indépendamment l'un de l'autre, un groupe alkyle ramifié ou non, éventuellement substitué par des halogènes, ou cycloalkyle,

deux des restes $R^1$, $R^2$ et $R^3$ pouvant en outre former un système cyclique avec l'atome de carbone auquel ils sont reliés, et

Hal représente un atome d'halogène,

par réaction d'halogénures d'alkyle de formule

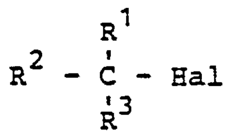

dans laquelle

$R^1$, $R^2$ et $R^3$ ont les significations indiquées ci-dessus,

avec l'oxyde de carbone sous pression, à température basse ou élevée, caractérisé en ce que l'on effectue la réaction en présence de 0,005 à 0,2 mole de chlorure d'aluminium et/ou de chlorure de fer par mole d'halogénure d'alkyle et le cas échéant en présence d'un autre acide de Brönsted ou de Lewis et le cas échéant en présence d'un solvant.

2. Procédé selon la revendication 1, caractérisé en ce que l'on utilise du chlorure d'aluminium.

3. Procédé selon les revendications 1 à 2, caractérisé en ce que l'on opère en présence de 20 à 500 % en volume d'un solvant par rapport au volume de l'halogénure d'alkyle.

4. Procédé selon les revendications 1 à 2, caractérisé en ce que l'on opère en présence de 50 à 250 % en volume d'un solvant.

5. Procédé selon les revendications 1 à 2, caractérisé en ce que l'on opère en présence de 80 à 150 % en volume d'un solvant.

6. Procédé selon les revendications 1 à 5, caractérisé en ce que l'on utilise en tant que solvant un solvant usuel dans les réactions de Friedel-Crafts et les réactions analogues.

7. Procédé selon les revendications 1 à 6, caractérisé en ce que l'on utilise en tant que solvant un hydrocarbure chloré.